(19) Europäisches Patentamt
European Patent Office
Office européen des brevets

(11) EP 4 541 425 A2

(12) EUROPEAN PATENT APPLICATION

(43) Date of publication:
**23.04.2025 Bulletin 2025/17**

(21) Application number: **25153814.6**

(22) Date of filing: **07.06.2023**

(51) International Patent Classification (IPC):
***A61P 35/02*** *(2006.01)*

(52) Cooperative Patent Classification (CPC):
(C-Sets available)
**A61K 31/17; A61K 31/122; A61K 31/69; A61K 45/06** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.06.2022 US 202263366004 P**

(62) Document number(s) of the earlier application(s) in accordance with Art. 76 EPC:
**23820426.7 / 4 536 365**

(71) Applicant: **Lantern Pharma Inc.**
**Dallas, TX 75201 (US)**

(72) Inventors:
- **BHATIA, Kishor**
**Dallas, TX 75201 (US)**
- **ZHOU, Jianli**
**Dallas, TX 75201 (US)**

(74) Representative: **Dr. Träger & Strautmann PAe PartG mbB**
**Stüvestraße 2**
**49076 Osnabrück (DE)**

Remarks:
This application was filed on 24-01-2025 as a divisional application to the application mentioned under INID code 62.

# (54) TREATING CANCERS WITH COMBINATIONS OF ACYLFULVENES WITH IBRUTINIB OR BORTEZOMIB

(57) A method of treating cancer includes a combination of a therapeutically effective amount of an illudin or an illudin analog thereof, derivative, or a pharmaceutically acceptable salt thereof; and a therapeutically effective amount of Bortezomib, Ibrutinib or an analog, derivative, or a pharmaceutically acceptable salt thereof. Compositions and kits of the same are included herein. The cancer may be refractory to Bortezomib and/or Ibrutinib.

A

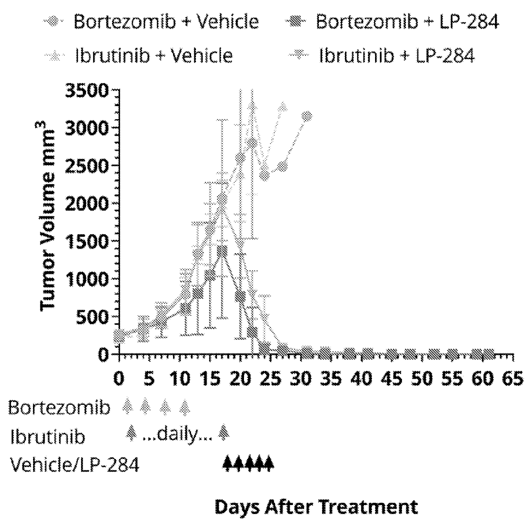


FIG. 2A

(52) Cooperative Patent Classification (CPC): (Cont.)

C-Sets
**A61K 31/122, A61K 2300/00;**
**A61K 31/17, A61K 2300/00;**
**A61K 31/69, A61K 2300/00**

## Description

TECHNICAL FIELD

This application relates to cancer treatments and more specifically this application relates to cancer treatments using a combination therapy including Ibrutinib and/or Bortezomib or a derivative thereof. More particularly, the combination treatment can be used to treat cancers that are refractory or relapsed subjects that were previously treated with Ibrutinib and/or Bortezomib.

BACKGROUND

Cancer is one of the most common causes of death in people. The development of therapeutic strategies for patients with advanced cancer has markedly improved overall survival. However, resistance to anticancer reagents is inevitable, and the prognosis of advanced cancer remains poor. There are several potential sources of cancer drug resistance, including alterations to drug transporters, the suppression of apoptosis, mitochondrial alterations, the promotion of DNA damage repair, autophagy, epithelial-mesenchymal transition, and cancer stem cells (CSCs). Appropriate strategies that consider the mechanisms are necessary to cure cancer.

Combination-therapy treatments for cancer have become more common, in part due to the perceived advantage of attacking the disease via multiple avenues. Although many effective combination-therapy treatments have been identified over the past few decades; in view of the continuing high number of deaths each year resulting from cancer, a continuing need exists to identify effective therapeutic regimens for use in anticancer treatment.

Accordingly, there is always a need for improved methods to treat cancer.

SUMMARY

This application discloses the discovery that treatment of a cancer in a subject with a combination of an illudin or illudin analog (e.g., acylfulvene) and Ibrutinib and/or Bortezomib (or derivative of the same) has greater effects than the effects provided by either acylfulvene or Ibrutinib and/or Bortezomib treatment, alone.

One aspect of this application includes a combination therapy for treating cancers. In embodiments, the therapy includes administering a combination of active agents including an illudin or illudin analog (e.g., acylfulvene), and Ibrutinib and/or Bortezomib. The methods include administering to a subject in need of treatment a combination of active agents having a therapeutically effective amount of an illudin or an illudin analog thereof, derivative, or a pharmaceutically acceptable salt thereof; and a therapeutically effective amount of Ibrutinib and/or Bortezomib or an analog, derivative, or a pharmaceutically acceptable salt thereof. The subject can have relapsed cancer and/or refractory cancer. The subject may have been treated previously with Ibrutinib and/or Bortezomib. The subject may have been treated with the combination after the cancer is refractory and resistant to Ibrutinib and/or Bortezomib.

One aspect includes a method in which the subject subsequently relapsed more than about 1 month, 2 months, 3 months, 4 months, or more following the cessation of treatment with Ibrutinib and/or Bortezomib.

Another aspect includes a method in which the illudin analog is an acylfulvene.

Another aspect includes a method in which the illudin analog is HydroxyUreaMethyl Acylfulvene.

Another aspect includes a method in which the illudin analog has the following structure:

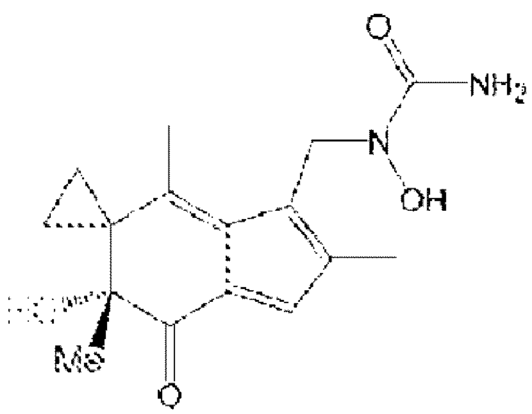

Another aspect includes a method in which the illudin analog has the following structure:

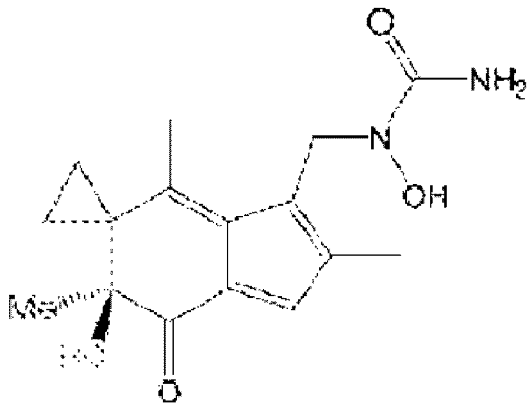

Another aspect includes a method in which the illudin analog is lrofulven.

Another aspect of this application provides pharmaceutical compositions comprising an illudin or illudin analog (e.g., acylfulvene) and Ibrutinib and/or Bortezomib or pharmaceutically acceptable salts thereof, mixed with pharmaceutically suitable carriers or excipient(s) at doses to treat or prevent cancer, primarily those who have been previously treated with Ibrutinib and/or Bortezomib. The pharmaceutical compositions can also be administered in combination with other therapeutic agents or therapeutic modalities simultaneously, sequentially, or in alternation.

Another aspect of this application includes the therapy including an acylfulvene that is (+) - hydroxyureamethyl acylfulvene.

Another aspect of this application includes the treatments of cancer that can include solid tumors, and hematological malignancies. Tumors such as, but not limited to, hyperplastic or neoplastic disease, such as a carcinoma, sarcoma, or mixed type cancer, including breast, colon, rectal, endometrial, gastric, prostate or brain, mesothelioma, ovarian, lung or pancreatic cancer can be targeted for therapy.

BRIEF DESCRIPTION OF THE FIGURES

FIG. 1 shows that cancer cells become refractory and relapse after days of treatment with Ibrutinib and/or Bortezomib;

FIG. 2A shows that tumor volumes were diminished rapidly in the LP-284 arms;

FTG. 2B shows a representative photo that shows large tumors in a mouse after switching vehicle/control treatment (upper) and near complete tumor regression in a mouse after switching to LP-284 treatment (lower); and

FIG. 2C shows Kaplan-Meier survival curves that demonstrate prolonged survival of the LP-284 rescued groups.

DETAILED DESCRIPTION

This application provides a combination therapy for treating solid cancers and blood cancers. In embodiments, the therapy includes administering a combination including an illudin or an illudin analog (e.g., acylfulvene) and Ibrutinib and/or Bortezomib, primarily, e.g., to those that have relapse or refractory cancers and that have been treated with Ibrutinib and/or Bortezomib. In other embodiments, the therapy includes administering a combination of other therapies. In other embodiments, the combination therapy can be used to treat biochemical occurrence or recurrence of solid cancers (e.g., lung cancer, breast cancer, ovarian cancer, prostate cancer, colon cancer, rectum cancer, and bladder cancer), glioblastoma and atypical teratoid rhabdoid, and renal cell carcinoma). In other embodiments, the therapy includes the combination therapy that can be used to treat biochemical occurrence and recurrence of blood cancers in which an acylfulvene (e.g., hydroxyureamethyl acylfulvene) or salt thereof and Ibrutinib and/or Bortezomib administered in a therapeutically effective amount to the patient. In certain embodiments, the combination can provide a treatment for lymphoma, such as mantle cell lymphoma (MCL) and double-hit lymphoma (DHL). In multiple myeloma (MM), the overgrowth of plasma cells in the bone marrow can crowd out normal blood-forming cells.

**Illudin or Acylfulvene**

In one embodiment, this application includes the use of an illudin or illudin analog (e g., acylfulvene). Acylfulvene

is a class of cytotoxic semi-synthetic derivatives of illudin, a natural product that can be extracted from the jack o'lantern mushroom (Omphalotus olearius). Acylfulvene, derived from the sesquiterpene illudin S by treatment with acid (reverse Prins reaction), is far less reactive to thiols than illudin S.

In one example, the acylfulvene is (-) - hydroxyureamethyl acylfulvene (termed LP-184 by Lantern Pharma Inc.), which shifts light negatively, is shown below:

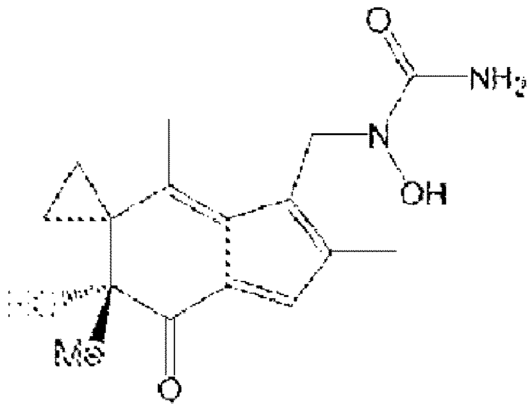

In another example, the acylfulvene is (+)-hydroxyureamethyl acylfulvene (termed LP-284 by Lantern Pharma Inc.), which shifts light positively, is shown below:

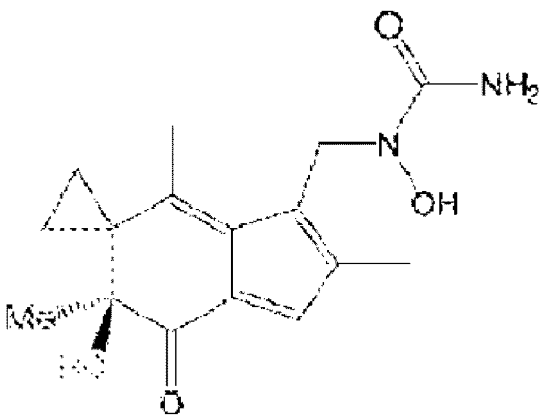

(+) - hydroxyureamethyl acylfulvene and (-) - hydroxyureamethyl acylfulvene are enantiomers and are now known publicly.

In another example, the acylfulvene is Irofulven.

## Bortezomib

Bortezomib is in a class of medications called antineoplastic agents. It works by killing cancer cells. Bortezomib, sold under the brand name Velcade among others, is an anticancer medication used to treat multiple myeloma and mantle cell lymphoma.

Bortezomib ([(1 R)-3-methyl-I-({(2S)-3-phenyl-2-[(pyrazin-2-ylcarbonyl)amino]propanoyl}amino)butyl]boronic acid) is a proteasome inhibitor having the structure shown below. Bortezomib may be obtained from Janssen. The term "Bortezomib" includes its pharmaceutically acceptable salts, solvates and hydrates thereof.

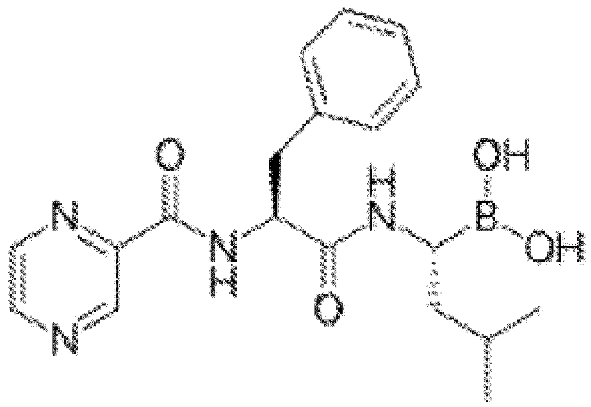

Stable liquid pharmaceutical compositions of bortezomib are described in WO2016/166653 and any such compositions may be used in the methods, compositions and uses of the invention.

In some embodiments, the composition comprising bortezomib is a "ready to use" formulation that contains bortezomib in dissolved or solubilized form and is intended to be used as such or upon further dilution in intravenous diluents. In preferred embodiments, pharmaceutical compositions comprising bortezomib are formulated for parenteral administration, e.g. injection or infusion.

Bortezomib may be administered in any suitable pharmaceutical form. For instance, bortezomib may be provided as a pharmaceutical composition comprising bortezomib or a salt, solvate or hydrate thereof together with a pharmacologically (or pharmaceutically) acceptable excipient.

Suitable solvents can be selected from aqueous and non-aqueous solvents such as, but are not limited to, glycerin, ethanol, n-propanol, n-butanol, isopropanol, ethyl acetate, dimethyl carbonate, acetonitrile, dichloromethane, methyl ethyl ketone, methyl isobutyl ketone, cyclohexane, dimethylacetamide (DMA), dimethyl sulfoxide (DMSO), N-methyl-2-pyrrolidone (NMP), I,3-dimethyl-2-imidazolidinone (DMT), acetone, tetrahydrofuran (THF), dimethylformamide (DMF), propylene carbonate (PC), dimethyl isosorbide, water and mixtures thereof. Preferred solvents are ethanol, glycerin and water.

The bortezomib formulation for use in the present invention may comprise stabilizers such as sugars and amino acids. Suitable stabilizers include glucose, trehalose, sucrose, mannitol, sorbitol, arginine, glycine, proline, methionine, lysine and the like.

The bortezomib formulation for use in the present invention may comprise a chelating agent. Suitable chelating agents include DOTA (1,4,7,10- tetraazacyclododecane-1,4,7,10-tetraacetic acid), DTPA (diethylene triaminepentaacetic acid), EDTA (Ethylenediaminetetraacetic acid), ODDA (1,4,10,13-tetraoxa-7,16- diazacyclooctadecane-7), TTT A (1,7,13 -triaza-4, 10,16- trioxacyclooctadecane-N,N',N" - triacetate), DOTRP (tetraethyleneglycol-1,5,9- triazacyclododecane-N,N',N",- tris(methylene phosphonic acid), EGTA (ethylene glycol-bis(P-aminoethyl ether)-tetraacetic acid) and the like.

The bortezomib formulation for use in the present invention may also contain one or more antioxidants. Suitable anti-oxidants include, but are not limited to monothioglycerol, ascorbic acid, sodium bisulfite, sodium metabisulfite, L-cysteine, thioglycolic acid, citric acid, tartaric acid, phosphoric acid, gluconic acid, thiodipropionic acid and the like. Most preferred anti-oxidant is monothioglycerol.

The most preferred aspect of administration of a combination of bortezomib and additional drugs for treatment of cancers is bortezomib in the form of a subcutaneous- or intravenous injection.

The bortezomib injection to be used according to the present invention is preferably in the form of a water-soluble boronic acid ester; the most preferably ester is a mannitol boronic acid ester.

The boronic acid ester formulation, preferably the mannitol ester, is typically in the form of a sterile dry powder formulation. The powder is typically a freeze dried powder. The powder is to be dissolved in sterile water, typically sterile isotonic aqueous sodium chloride solution before administration.

The bortezomib formulation for use in the present invention may optionally contain other pharmaceutically acceptable adjuvants such as buffering agents, pH adjusting agents, preservatives, tonicity modifiers and the like. The lists of solvents, stabilizers, chelating agents and antioxidants listed above may also be used in pharmaceutical compositions comprising other cytotoxic agents described herein unless stated otherwise.

The bortezomib-based formulation described above might preferably comprise mannitol and might be provided in an injection vial under a nitrogen atmosphere or in a prefilled syringe.

**Bruton's tyrosine kinase (BTK) Inhibitor Compounds Including Ibrutinib, and Pharmaceutically Acceptable Salts Thereof**

In some embodiments, the Btk inhibitor compounds described herein are selective for Btk and kinases having a cysteine residue in an amino acid sequence position of the tyrosine kinase that is homologous to the amino acid sequence position of cysteine 481 in Btk. The Btk inhibitor compounds can form a covalent bond with Cys 481 of Btk (e.g., via a Michael reaction).

In some embodiments, the Btk inhibitor is (R)-I-(3-(4-amino-3-(4-phenoxyphenyl)-1H-pyrazolo[3,4-d]pyrimidin-1-yl)piperidin-1-yl)prop-2-en-1-one (i.e. PCI-32765/ibrutinib).

Another embodiment includes a pharmaceutical composition having a therapeutically effective amount of an illudin or an illudin analog thereof, derivative, or a pharmaceutically acceptable salt thereof; and a therapeutically effective amount of Ibrutinib and/or Bortezomib or an analog, derivative, or a pharmaceutically acceptable salt thereof. The illudin analog can be HydroxyUreaMethylAcylfulvene.

In another embodiment, a kit for the treatment of cancer in a subject includes a therapeutically effective amount of an illudin or an illudin analog thereof, derivative, or a pharmaceutically acceptable salt thereof; and a therapeutically effective amount of Ibrutinib and/or Bortezomib or an analog, derivative, or a pharmaceutically acceptable salt thereof:

In another embodiment, the second therapeutic is one or more chemotherapeutic agents selected from camptothecin derivatives, paclitaxel, docetaxel, epothilone B, 5-FU, gemcitabine, oxaliplatin, cisplatinum, carboplatin, melphalam, dacarbazine, temozolomide, doxorubicin, imatinib, erlotinib, bevacizumab, cetuximab and a Raf kinase inhibitor.

In another embodiment, the second therapeutic is one or more chemotherapeutic agents selected from paclitaxel or cisplatinum.

The term "combination therapy" can include or includes the administration of the therapeutic agents as described above in further combination with other biologically active ingredients and non-drug therapies (e.g., surgery or radiation treatment). Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks or months.

In another aspect, a composition or combination therapy herein, or a pharmaceutically acceptable salt or solvate thereof, may be administered in combination with radiation therapy. Radiation therapy can also be administered in combination with a composition of the present invention and another chemotherapeutic agent described herein as part of a multiple agent therapy.

Combination therapy can be achieved by administering two or more agents, e.g., an acylfulvene, Ibrutinib and/or Bortezomib and one or more other therapeutic agents, each of which is formulated and administered separately, or by administering two or more agents in a single formulation. Other combinations are also encompassed by combination therapy. For example, two agents can be formulated together and administered in conjunction with a separate formulation containing a third agent. While the two or more agents in the combination therapy can be administered simultaneously, they need not be. For example, administration of a first agent (or combination of agents) can precede administration of a second agent (or combination of agents) by minutes, hours, days, or weeks. Thus, the two or more agents can be administered within minutes of each other or within 1, 2, 3, 6, 9, 12, 15, 18, or 24 hours of each other or within 1, 2, 3, 4, 5, 6, 7, 8, 9, 10, 12, 14 days of each other or within 2, 3, 4, 5, 6, 7, 8, 9, or 10 weeks of each other. In some cases, even longer intervals are possible. While in many cases it is desirable that the two or more agents used in a combination therapy be present within the patient's body at the same time, this need not be so.

The methods of combination therapy may or should result in a synergistic effect, wherein the effect of a combination of compounds or other therapeutic agents is greater than the sum of the effects resulting from administration of any of the compounds or other therapeutic agents as single agents. A synergistic effect may also be an effect that cannot be achieved by administration of any of the compounds or other therapeutic agents as single agents. The synergistic effect may include, but is not limited to, an effect of treating cancer by reducing tumor size, inhibiting tumor growth, or increasing survival of the subject. The synergistic effect may also include reducing cancer cell viability, inducing cancer cell death, and inhibiting or delaying cancer cell growth.

Therapeutically effective doses can vary, as recognized by those skilled in the art, depending on the diseases treated, the severity of the disease, the route of administration, the age and general health condition of the patient, excipient usage, the possibility of co-usage with other therapeutic treatments such as use of other agents and the judgment of the treating physician. For example, guidance for selecting an effective dose can be determined by reference to the prescribing information for acylfulvene or hydroxyureamethyl acylfulvene or journal discussion the same.

The term "effective amount" as used herein refers to the amount of an agent needed to alleviate at least one or more symptoms of the disease or disorder, and relates to a sufficient amount of pharmacological composition to provide

the desired effect. The term "therapeutically effective amount" therefore refers to an amount of the agent that is sufficient to provide a particular effect when administered to a typical subject. An effective amount may be an amount sufficient to decrease the symptoms of a disease responsive to inhibition of Ibrutinib and/or Bortezomib. For cancer therapy, efficacy in vivo can, for example, be measured by assessing the duration of survival, time to disease progression (TTP), the response rates (RR), duration of response, and/or quality of life. Effective amounts may vary, as recognized by those skilled in the art, depending on route of administration, excipient usage, and co-usage with other agents. An effective amount as used herein, in various contexts, would also include an amount sufficient to delay the development of a symptom of the disease, alter the course of a symptom disease (for example but not limited to, slowing the progression of a symptom of the disease), or reverse a symptom of the disease. Thus, it is not generally practicable to specify an exact "effective amount". However, for any given case, an appropriate "effective amount" can be determined by one of ordinary skill in the art using only routine experimentation.

The dosage ranges for the administration of an agent according to the methods described herein depend upon, for example, the form of the agent, its potency, and the extent to which symptoms, markers, or indicators of a condition described herein are desired to be reduced, for example, the percentage reduction desired for tumor growth. The dosage should not be so large as to cause adverse side effects. Generally, the dosage will vary with the age, condition, and sex of the patient and can be determined by one of skill in the art. The dosage can also be adjusted by the individual physician in the event of any complication.

The term "therapeutically effective amount", as used herein, refers to an amount of a pharmaceutical agent to treat, ameliorate, or prevent an identified disease or condition, or to exhibit a detectable therapeutic or inhibitory effect. The effect can be detected by any assay method known in the art. The precise effective amount for a subject will depend upon the subject’s body weight, size, and health; the nature and extent of the condition; and the therapeutic or combination of therapeutics selected for administration. Therapeutically effective amounts for a given situation can be determined by routine experimentation that is within the skill and judgment of the clinician. In a preferred aspect, the disease or condition to be treated is cancer. In another aspect, the disease or condition to be treated is a cell proliferative disorder.

The efficacy of an agent described herein in, e.g., the treatment of a condition described herein, or to induce a response as described herein (e.g., solid cancers or blood cancers) can be determined by the skilled clinician. However, a treatment is considered "effective treatment," as the term is used herein, if one or more of the signs or symptoms of a condition described herein are altered in a beneficial manner, other clinically accepted symptoms are improved, or even ameliorated, or a desired response is induced e.g., by at least 10% following treatment according to the methods described herein. Efficacy can be assessed, for example, by measuring a marker, indicator, symptom, and/or the incidence of a condition treated according to the methods described herein or any other measurable parameter appropriate, e.g. tumor size and/or growth rate. Efficacy can also be measured by a failure of an individual to worsen as assessed by hospitalization, or need for medical interventions (i.e., progression of the disease is halted). Methods of measuring these indicators are known to those of skill in the art and/or are described herein. Treatment includes any treatment of a disease in an individual or an animal (some non-limiting examples include a human or an animal) and includes: (1) inhibiting the disease, e.g., preventing a worsening of symptoms (e.g., pain or inflammation); or (2) relieving the severity of the disease, e.g., causing regression of symptoms. An effective amount for the treatment of a disease means that amount which, when administered to a subject in need thereof, is sufficient to result in effective treatment as that term is defined herein, for that disease. Efficacy of an agent can be determined by assessing physical indicators of a condition or desired response. It is well within the ability of one skilled in the art to monitor efficacy of administration and/or treatment by measuring any one of such parameters, or any combination of parameters. Efficacy can be assessed in animal models of a condition described herein, for example, treatment of blood cancers in a mouse model. When using an experimental animal model, efficacy of treatment is evidenced when a statistically significant change in a marker is observed, e.g. tumor size and/or growth rate. In some embodiments, the therapeutically effective amount of hydroxyureamethyl-acylfulvene, acylfulvene, Irofulven or a pharmaceutically acceptable salt thereof is selected from the group consisting of 0.5 mg/day, 1 mg/day, 2.5 mg/day, 5 mg/day, 10 mg/day, 20 mg/day, 30 mg/day, 60 mg/day, 90 mg/day, 120 mg/day, 150 mg/day, 180 mg/day, 210 mg/day, 240 mg/day, 270 mg/day, 300 mg/day, 360 mg/day, 400 mg/day, 440 mg/day, 480 mg/day, 520 mg/day 580 mg/day, 600 mg/day, 620 mg/day, 640 mg/day, 680 mg/day, and 720 mg/day.

The administration dose should be adjusted for the requirement of the individual in need For example, the administered dosage of Ibrutinib and/or Bortezomib is 1-120 mg (in terms of the parent compound), preferably, 1-80 mg (in terms of the parent compound), and the administration frequency is twice a day (BID); the administered dosage of Ibrutinib and/or Bortezomib is 1-120-240 mg (in terms of the parent compound), preferably, 60-120 mg (in terms of the parent compound), and the administration frequency is once a day (QD). In some cases, it is more suitable to apply the lower end of the above described dosage ranges, while in other cases the higher dosages may be used without causing harmful side effects.

The typical dose of ibrutinib varies depending on the medical condition being treated. In most cases, the starting dose for adult patients is 420 mg once daily, while for pediatric patients, it can range from 160 to 440 mg depending on their height and weight. However, the dose may be adjusted based on individual patient response and tolerance.

The term "treat" is used and includes both therapeutic treatment and prophylactic treatment (reducing the likelihood of development) Both terms mean decrease, suppress, attenuate, diminish, arrest, or stabilize the development or progression of a disease (e.g., a disease or disorder delineated herein), lessen the severity of the disease or improve the symptoms associated with the disease.

The typical dose of bortezomib depends on the medical condition being treated and the patient's body weight. For multiple myeloma and mantle cell lymphoma, the recommended dose is 1.3 mg/m2 two times a week for two weeks, followed by a 10-day rest period. This 21- day cycle is repeated for up to eight cycles. However, the dosage may be adjusted based on the individual's response and tolerance to the treatment.

The pharmaceutical compositions can be included in a container, pack, or dispenser together with instructions for administration.

The composition of the present invention is capable of further forming salts. The composition of the present invention can form more than one salt per molecule, e.g., mono-, di-, tri-. All of these forms are also contemplated within the scope of the claimed invention.

As used herein, "pharmaceutically acceptable salts" refer to derivatives of the compounds of the present invention wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

Other examples of pharmaceutically acceptable salts include hexanoic acid, cyclopentane propionic acid, pyruvic acid, malonic acid, 3-(4-hydroxybenzoyl)benzoic acid, cinnamic acid, 4-chlorobenzenesulfonic acid, 2-naphthalenesulfonic acid, 4-toluenesulfonic acid, camphorsulfonic acid, 4-methylbicyclo-[2.2.2]-oct-2-ene-1-carboxylic acid, 3-phenylpropionic acid, trimethylacetic acid, tertiary butylacetic acid, muconic acid, and the like. The present invention also encompasses salts formed when an acidic proton in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like.

It should be understood that all references to pharmaceutically acceptable salts include solvent addition forms (solvates), of the same salt.

As used herein, the term "selectively" means tending to occur at a higher frequency in one population than in another population. The compared populations can be cell populations. Preferably, an event occurs selectively in population A relative to population B if it occurs greater than two times more frequently in population A as compared to population B. An event occurs selectively if it occurs greater than five times more frequently in population A. An event occurs selectively if it occurs greater than ten times more frequently in population A; more preferably, greater than fifty times; even more preferably, greater than 100 times; and most preferably, greater than 1000 times more frequently in population A as compared to population B. For example, cell death would be said to occur selectively in cancer cells if it occurred greater than twice as frequently in cancer cells as compared to normal cells.

The composition, or pharmaceutically acceptable salts or solvates thereof, are administered orally, nasally, transdermally, pulmonary, inhalationally, buccally, sublingually, intraperintoneally, subcutaneously, intramuscularly, intravenously, rectally, intrapleurally, intrathecally and parenterally. In one embodiment, the compound is administered orally. One skilled in the art will recognize the advantages of certain routes of administration.

The dosage regimen utilizing the compounds is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Techniques for formulation and administration of the disclosed compounds of the invention can be found in Remington: the Science and Practice of Pharmacy, 19.sup.th edition, Mack Publishing Co., Easton, Pa. (1995). In an embodiment, the compounds described herein, and the pharmaceutically acceptable salts thereof, are used in pharmaceutical preparations in combination with a pharmaceutically acceptable carrier or diluent. Suitable pharmaceutically acceptable carriers include inert solid fdlers or diluents and sterile aqueous or organic solutions. The compounds will be present in such pharmaceutical compositions in amounts sufficient to provide the desired dosage amount in the range

described herein.

All percentages and ratios used herein, unless otherwise indicated, are by weight. Other features and advantages of the present invention are apparent from the different examples. The provided examples illustrate different components and methodology useful in practicing the present invention. The examples do not limit the claimed invention. Based on the present disclosure the skilled artisan can identify and employ other components and methodology useful for practicing the present invention.

As used herein, a "subject in need thereof' is a subject having a precancerous condition. Preferably, a subject in need thereof has cancer. A "subject" includes a mammal. The mammal can be e.g., any mammal, e.g., a human, primate, bird, mouse, rat, dog, cat, cow, horse, goat, camel, sheep or a pig. Preferably, the mammal is a human. The subject of the present invention includes any human subject who has been diagnosed with, has symptoms of, or is at risk of developing a cancer or a precancerous condition.

A subject in need thereof may have refractory or resistant cancer. "Refractory or resistant cancer" means cancer that does not respond to treatment. The cancer may be resistant at the beginning of treatment or it may become resistant during treatment. In some embodiments, the subject in need thereof has cancer recurrence following remission on most recent therapy. In some embodiments, the subject in need thereof received and failed all known effective therapies for cancer treatment. In some embodiments, the subject in need thereof received at least one prior therapy. In certain embodiments the prior therapy is monotherapy. In certain embodiments the prior therapy is combination therapy.

A "relapsed cancer" is a cancer that has previously been treated and, as a result of that treatment, the subject made a complete or partial recovery (i.e. the subject is said to be in remission), but that after the cessation of the treatment the cancer returned or worsened. Put another way, a relapsed cancer is one that has become resistant to a treatment, after a period in which it was effective and the subject made a complete or partial recovery. Typically, in patients with multiple myeloma, the development of resistance and refractory disease occurs after multiple rounds of treatment. Further, Ibrutinib can become refractory when the cancer cells develop mutations or alternate signaling pathways that bypass the drug's mechanism of action, leading to treatment resistance. This can happen at various stages of treatment and may vary depending on the type of cancer being treated and the patient's individual response to the drug.

In some embodiments, a subject in need thereof may have a secondary cancer as a result of a previous therapy. "Secondary cancer" means cancer that arises due to or as a result from previous carcinogenic therapies, such as chemotherapy.

Cancer is a group of diseases that may cause almost any sign or symptom. The signs and symptoms will depend on where the cancer is, the size of the cancer, and how much it affects the nearby organs or structures. If a cancer spreads (metastasizes), then symptoms may appear in different parts of the body.

Treating cancer can result in a reduction in size of a tumor. A reduction in size of a tumor may also be referred to as "tumor regression". Preferably, after treatment, tumor size is reduced by 5% or greater relative to its size prior to treatment; more preferably, tumor size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75% or greater. Size of a tumor may be measured by any reproducible means of measurement. The size of a tumor may be measured as a diameter of the tumor.

Treating cancer results in a decrease in number and size of tumors. Preferably, after treatment, tumor number or size is reduced by 5% or greater relative to number prior to treatment; more preferably, tumor number or size is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. Number of tumors may be measured by any reproducible means of measurement. The number of tumors may be measured by counting tumors visible to the naked eye or at a specified magnification. Preferably, the specified magnification is 2x, 3x, 4x, 5x, 10x, or 50x.

Treating cancer can result in a decrease in number of metastatic lesions in other tissues or organs distant from the primary tumor site. Preferably, after treatment, the number of metastatic lesions is reduced by 5% or greater relative to number prior to treatment; more preferably, the number of metastatic lesions is reduced by 10% or greater; more preferably, reduced by 20% or greater; more preferably, reduced by 30% or greater; more preferably, reduced by 40% or greater; even more preferably, reduced by 50% or greater; and most preferably, reduced by greater than 75%. The number of metastatic lesions may be measured by any reproducible means of measurement. The number of metastatic lesions may be measured by counting metastatic lesions visible to the naked eye or at a specified magnification. Preferably, the specified magnification is 2x, 3x, 4x, 5x, 10x, or 50x.

Treating cancer can result in an increase in average survival time of a population of treated subjects in comparison to a population receiving carrier alone. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population

may also be measured, for example, by calculating for a population the average length of survival following completion of a first round of treatment with an active compound.

Treating cancer can result in an increase in average survival time of a population of treated subjects in comparison to a population of untreated subjects. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer can result in increase in average survival time of a population of treated subjects in comparison to a population receiving monotherapy with a drug that is not a compound of the present invention, or a pharmaceutically acceptable salt or solvate thereof. Preferably, the average survival time is increased by more than 30 days; more preferably, by more than 60 days; more preferably, by more than 90 days; and most preferably, by more than 120 days. An increase in average survival time of a population may be measured by any reproducible means. An increase in average survival time of a population may be measured, for example, by calculating for a population the average length of survival following initiation of treatment with an active compound. An increase in average survival time of a population may also be measured, for example, by calculating for a population the average length of survival following completion of a first round of treatment with an active compound.

Treating cancer can result in a decrease in the mortality rate of a population of treated subjects in comparison to a population receiving carrier alone. Treating cancer can result in a decrease in the mortality rate of a population of treated subjects in comparison to an untreated population. Treating cancer can result in a decrease in the mortality rate of a population of treated subjects in comparison to a population receiving monotherapy with a drug that is not a compound of the present invention, or a pharmaceutically acceptable salt or solvate thereof. Preferably, the mortality rate is decreased by more than 2%; more preferably, by more than 5%; more preferably, by more than 10%; and most preferably, by more than 25%. A decrease in the mortality rate of a population of treated subjects may be measured by any reproducible means. A decrease in the mortality rate of a population may be measured, for example, by calculating for a population the average number of disease-related deaths per unit time following initiation of treatment with an active compound. A decrease in the mortality rate of a population may also be measured, for example, by calculating for a population the average number of disease-related deaths per unit time following completion of a first round of treatment with an active compound.

Treating cancer can result in a decrease in tumor growth rate. Preferably, after treatment, tumor growth rate is reduced by at least 5% relative to number prior to treatment; more preferably, tumor growth rate is reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Tumor growth rate may be measured by any reproducible means of measurement. Tumor growth rate can be measured according to a change in tumor diameter per unit time.

Treating cancer can result in a decrease in tumor regrowth. Preferably, after treatment, tumor regrowth is less than 5%; more preferably, tumor regrowth is less than 10%; more preferably, less than 20%; more preferably, less than 30%; more preferably, less than 40%; more preferably, less than 50%; even more preferably, less than 50%; and most preferably, less than 75%. Tumor regrowth may be measured by any reproducible means of measurement. Tumor regrowth is measured, for example, by measuring an increase in the diameter of a tumor after a prior tumor shrinkage that followed treatment. A decrease in tumor regrowth is indicated by failure of tumors to reoccur after treatment has stopped.

Treating or preventing a cell proliferative disorder can result in a reduction in the rate of cellular proliferation. Preferably, after treatment, the rate of cellular proliferation is reduced by at least 5%; more preferably, by at least 10%; more preferably, by at least 20%; more preferably, by at least 30%; more preferably, by at least 40%; more preferably, by at least 50%; even more preferably, by at least 50%; and most preferably, by at least 75%. The rate of cellular proliferation may be measured by any reproducible means of measurement. The rate of cellular proliferation is measured, for example, by measuring the number of dividing cells in a tissue sample per unit time.

Treating or preventing a cell proliferative disorder can result in a reduction in the proportion of proliferating cells. Preferably, after treatment, the proportion of proliferating cells is reduced by at least 5%; more preferably, by at least 10%; more preferably, by at least 20%; more preferably, by at least 30%; more preferably, by at least 40%; more preferably, by at least 50%; even more preferably, by at least 50%, and most preferably, by at least 75%. The proportion of proliferating cells may be measured by any reproducible means of measurement. Preferably, the proportion of proliferating cells is measured, for example, by quantifying the number of dividing cells relative to the number of nondividing cells in a tissue sample. The proportion of proliferating cells can be equivalent to the mitotic index.

Treating or preventing a cell proliferative disorder can result in a decrease in size of an area or zone of cellular proliferation. Preferably, after treatment, size of an area or zone of cellular proliferation is reduced by at least 5% relative to

its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. Size of an area or zone of cellular proliferation may be measured by any reproducible means of measurement. The size of an area or zone of cellular proliferation may be measured as a diameter or width of an area or zone of cellular proliferation.

Treating or preventing a cell proliferative disorder can result in a decrease in the number or proportion of cells having an abnormal appearance or morphology. Preferably, after treatment, the number of cells having an abnormal morphology is reduced by at least 5% relative to its size prior to treatment; more preferably, reduced by at least 10%; more preferably, reduced by at least 20%; more preferably, reduced by at least 30%; more preferably, reduced by at least 40%; more preferably, reduced by at least 50%; even more preferably, reduced by at least 50%; and most preferably, reduced by at least 75%. An abnormal cellular appearance or morphology may be measured by any reproducible means of measurement. An abnormal cellular morphology can be measured by microscopy, e.g., using an inverted tissue culture microscope. An abnormal cellular morphology can take the form of nuclear pleiomorphism.

Administering a composition of the present invention to a cell or a subject in need thereof can result in modulation (i.e., stimulation or inhibition) of an activity of a protein methyltransferase of interest.

Treating cancer or a cell proliferative disorder can result in cell death, and preferably, cell death results in a decrease of at least 10% in number of cells in a population. More preferably, cell death means a decrease of at least 20%; more preferably, a decrease of at least 30%; more preferably, a decrease of at least 40%; more preferably, a decrease of at least 50%, most preferably, a decrease of at least 75%. Number of cells in a population may be measured by any reproducible means. A number of cells in a population can be measured by fluorescence activated cell sorting (FACS), immunofluorescence microscopy and light microscopy. Methods of measuring cell death are as shown in Li et al., Proc. Natl. Acad. Sci. USA. 100(5): 2674-8, 2003. In an aspect, cell death occurs by apoptosis.

Preferably, an effective amount of a composition of the present invention, or a pharmaceutically acceptable salt or solvate thereof, is not significantly cytotoxic to normal cells. A therapeutically effective amount of a compound is not significantly cytotoxic to normal cells if administration of the compound in a therapeutically effective amount does not induce cell death in greater than 10% of normal cells. A therapeutically effective amount of a compound does not significantly affect the viability of normal cells if administration of the compound in a therapeutically effective amount does not induce cell death in greater than 10% of normal cells. In an aspect, cell death occurs by apoptosis.

Contacting a cell with a composition of the present invention, or a pharmaceutically acceptable salt or solvate thereof, can induce, or activate cell death selectively in cancer cells. Administering to a subject in need thereof a compound of the present invention, or a pharmaceutically acceptable salt or solvate thereof, can induce or activate cell death selectively in cancer cells. Contacting a cell with a composition of the present invention, or a pharmaceutically acceptable salt or solvate thereof, can induce cell death selectively in one or more cells affected by a cell proliferative disorder. Preferably, administering to a subject in need thereof a composition of the present invention, or a pharmaceutically acceptable salt or solvate thereof, induces cell death selectively in one or more cells affected by a cell proliferative disorder.

The present invention relates to a method of treating or preventing cancer by administering a composition of the present invention, or a pharmaceutically acceptable salt or solvate thereof, to a subject in need thereof, where administration of the composition of the present invention, or a pharmaceutically acceptable salt or solvate thereof, results in one or more of the following: prevention of cancer cell proliferation by accumulation of cells in one or more phases of the cell cycle (e.g. G1, G1/S, G2/M), or induction of cell senescence, or promotion of tumor cell differentiation; promotion of cell death in cancer cells via cytotoxicity, necrosis or apoptosis, without a significant amount of cell death in normal cells, antitumor activity in animals with a therapeutic index of at least 2. As used herein, "therapeutic index" is the maximum tolerated dose divided by the efficacious dose.

The term "kit" means a combination partner as defined above can be dosed independently or by use of different fixed combinations with distinguished amounts of the combination partners, i.e. simultaneously or at different time points. The parts of the kit of parts can then, e.g., be administered simultaneously or chronologically staggered, that is at different time points and with equal or different time intervals for any part of the kit of parts. The ratio of the total amounts of the combination partners to be administered in the combined preparation can be varied. The combination partners can be administered by the same route or by different routes.

One skilled in the art may refer to general reference texts for detailed descriptions of known techniques discussed herein or equivalent techniques. These texts can, of course, also be referred to in making or using an aspect of the invention.

## EXAMPLES

In order that the disclosure disclosed herein may be more efficiently understood, examples are provided below. It should be understood that these examples are for illustrative purposes only and are not to be construed as limiting the disclosure in any manner.

Example 1

[88] The effect of LP-284 and Ibrutinib and/or Bortezomib on the growth of MCL xenograft tumors is shown in FIGs. 1 and 2 A. The JeKo-1 MCL cell line-derived xenograft mice (n = 8/arm) were treated with vehicle, LP-284 (2 mg/kg or 4 mg/kg, i.v.), bortezomib (1 mg/kg, i.p.), or ibrutinib (50 mg/kg, p.o.) at days indicated by arrows in the graph. Tumor volumes were measured every 2 to 4 days after treatment initiation. FIG. 1 shows that cancer cells become refractory and relapse after days of treatment. Low dose LP-284, Ibrutinib and/or Bortezomib treatments each resulted in refractory cancer after initially controlling cell growth in tumors.

As shown in FIGs. 2A and 2C in the same study, LP-284 is capable of almost completely eliminating those lymphomas that are as large as 2000 mm3 and refractory to bortezomib or ibrutinib (FIG. 2A). There are numerous resistant mechanisms of these two drugs such as mutation and deregulation of the proteasome subunits, BTK mutation, upregulated NF-KB and cell survival pathways. These results show that LP-284 treatment salvages treatment plans for patients who are refractory to bortezomib or ibrutinib.

Example 2

FIGs. 2A, 2B, and 2C show that LP-284 with bortezomib or ibrutinib diminishes bortezomib-refractory and ibrutinib-refractory MCL xenograft tumors. JeKo-1 MCL xenograft tumors were refractory to bortezomib and ibrutinib under the dosage regimens as shown in FIG. 2A, leading to tumor volumes of ~ 2000 mm3 at day 17. The two drugs were replaced with one cycle of 4 mg/kg LP-284 or the vehicle (saline). Bortezomib and ibrutinib refractory mice were stratified based on their tumor size into two groups of four mice each that were then treated by LP-284 or the vehicle. Whereas the vehicle treated tumors continued expansion till all the mice were dead or sacrificed due to excess tumor size, one cycle of 4 mg/kg LP-284 led to near complete tumor regression (FIGs. 2A and 2B). In addition, LP-284 resulted in approximately two times longer survival ($p<0.001$) than vehicles in these bortezomib or ibrutinib pre-treated and refractory xenograft mice (Figure 2C). No significant weight loss at the end of treatment (EOT) was observed in any of the treatment arms (one-tailed T-test, $p>0.05$).

LP-284 rescues bortezomib and ibrutinib-refractory MCL xenograft mice. The JeKo-1 MCL cell line-derived xenograft mice were treated by bortezomib (1 mg/kg, i.p.) or ibrutinib (50 mg/kg, p.o.) till day 16 when the tumor sizes reached ~ 2000 mm3. Each treatment arm was then further split into two sub-arms (n = 4/sub-arm), one of which was treated by vehicle whereas the other was treated by 4 mg/kg LP-284 (i.v.), as indicated by arrows in the graph. FIG. 2A shows that tumor volumes were diminished rapidly in the LP-284 arms. FIG. 2B shows a representative photo that shows large tumors in a mouse after switching vehicle treatment (upper) and near complete tumor regression in a mouse after switching to LP-284 treatment (lower). FIG. 2C shows Kaplan-Meier survival curves indicating prolonged survival of the LP-284 rescued groups.

**Methodology**

**In vivo animal study**

Five-week old NOD.Scid mice were procured through Envigo (Item 17003F). Mice were fed Teklad irradiated (sterilized) mouse diet and bedded with Teklad irradiated (sterilized) corncob bedding from Envigo (Indianapolis, IN). Mice were housed in Optimice carousel sterile quarters with filtered air supply in disposable cages from Animal Care Systems, Inc. (Centennial, CO). A total of 1 x 107 cells JeKo-1 cells were injected subcutaneously into the right hind flank of each animal using a 27 gram needle. Vehicle (saline), 4 mg/kg or 2 mg/kg LP-284 was administered as an intravenous injection on day I, 3, 5, 7, 9, 17, 19, 21, 23, 25. 50 mg/kg ibrutinib was administered orally daily on day 1-16. 1 mg/kg bortezomib administered as an intraperitoneal injection on day 1, 4, 7, 10. The ibrutinib and bortezomib arms were further treated with vehicle or 4 mg/kg LP-284 intravenously on day 17, 19, 21, 23, 25. Tumors were measured in two dimensions using calipers, and volume was calculated using the formula:

$$\text{TumorVolume}(mm^3) = {}^{w2 \times l\ /}\ 2$$

where w = width and l = length, in mm, of the tumor. For this study the calipers were aligned to the tumor edges (the tumors were not squeezed with the caliper).

While a number of exemplary aspects and embodiments have been discussed above, those of skill in the art will recognize certain modifications, permutations, additions, and subcombinations thereof. It is therefore intended that the following appended claims and claims hereafter are interpreted to include all such modifications, permutations, additions, and subcombinations as are within their true spirit and scope.

**Further Embodiments of the invention**

Particular embodiments of the invention are disclosed below, in particular specific embodiments in each of the following paragraphs. Selections and combinations of individual features throughout the application are also possible, in particular in view of and with reference to the parent application.

A method of treating cancer, the method comprises administering to a subject in need of treatment a combination comprising:

a. a therapeutically effective amount of an illudin or an illudin analog thereof, derivative, or a pharmaceutically acceptable salt thereof; and
b. a therapeutically effective amount of Bortezomib or an analog, derivative, or a pharmaceutically acceptable salt thereof,

wherein the subject has relapsed cancer and/or refractory cancer; and the subject has been treated previously with Bortezomib; and the subject is treated with the combination after the cancer is refractory and resistant to Bortezomib.

The method discussed above, wherein further the subject subsequently relapsed more than about 1 month following the cessation of treatment with Bortezomib.

The method discussed above, wherein further the illudin analog is an acylfulvene.

The method discussed above, wherein further the illudin analog is HydroxyUreaMethyl Acylfulvene.

The method discussed above, wherein further the illudin analog has the following structure:

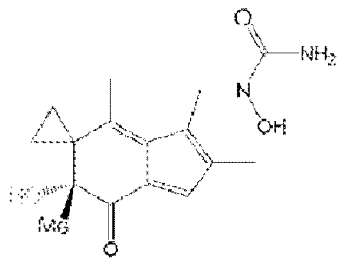

.

The method discussed above, wherein further the illudin analog has the following structure:

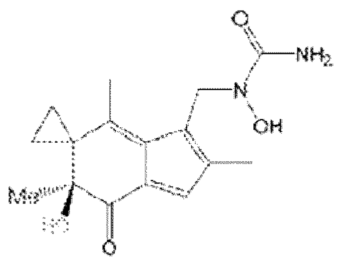

.

The method discussed above, wherein further the illudin analog is Irofulven.

The method discussed above, wherein further the Bortezomib is administered after at least two rounds of Bortezomib treatements.

The method discussed above, wherein further the bortezomib or a pharmaceutically acceptable salt, solvate or hydrate thereof and the illudin or an illudin analog are for separate, simultaneous or sequential use or administration.

The method discussed above, wherein further the Bortezomib is administrated at a dose of 160 to 440 mg daily.

The method discussed above, wherein further the active agents are administered separately.

The method discussed above, wherein further the active agents are administered daily.

The method discussed above, wherein further the active agents are administered sequentially.

The method discussed above, wherein further the active agents are administered as a coformulation.

The method discussed above, wherein further an illudin or an analog thereof administration is before, during, or after Bortezomib administration.

The method discussed above, wherein further the method further comprising administering radiotherapy, chemotherapy to, performing surgery on, the subject before, during, or following the illudin and/or administering the Bortezomib.

The method discussed above, wherein further the cancer is MCL.

The method discussed above, wherein further the subject is an animal.

The method discussed above, wherein further the subject or mammal is a human.

The method discussed above, further comprising subjecting the subject to radiation therapy before, after, or during treatment with HydroxyUreaMethyl Acylfulvene.

The method discussed above, further comprising administering an additional therapeutic agent selected from the group consisting of cisplatin, paclitaxel, and other available therapies.

The method discussed above, wherein further the cancer comprises a solid tumor.

The method discussed above, wherein further the cancer is lymphoma, leukemia, or melanoma.

A pharmaceutical composition comprising a therapeutically effective amount of an illudin or an illudin analog thereof, derivative, or a pharmaceutically acceptable salt thereof; and a therapeutically effective amount of Ibrutinib or an analog, derivative, or a pharmaceutically acceptable salt thereof.

The pharmaceutical composition discussed above, wherein further the illudin analog is HydroxyUreaMethyl Acylfulvene.

The pharmaceutical composition discussed above, wherein further the illudin analog has the following structure:

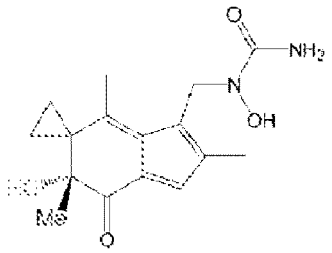

.

The pharmaceutical composition discussed above, wherein further the illudin analog has the following structure:

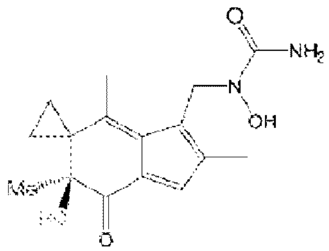

.

The pharmaceutical composition discussed above, wherein further the illudin analog is Irofulven.

A kit for the treatment of cancer in a subject comprising a therapeutically effective amount of an illudin or an illudin analog thereof, derivative, or a pharmaceutically acceptable salt thereof; and a therapeutically effective amount of Ibrutinib or an analog, derivative, or a pharmaceutically acceptable salt thereof, wherein the subject has relapsed cancer and/or refractory cancer; and the subject has been treated previously with the Ibrutinib; and the subject is treated with the combination after the cancer is refractory and resistant to Ibrutinib.

The pharmaceutical composition discussed above, wherein further the illudin analog is HydroxyUreaMethyl Acylfulvene.

The pharmaceutical composition discussed above, wherein further the illudin analog has the following structure:

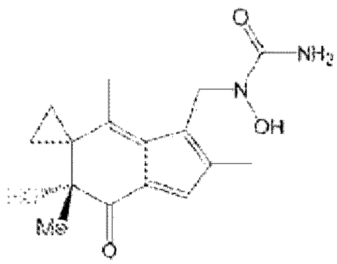

.

The pharmaceutical composition discussed above, wherein further the illudin analog is Irofulven.

The pharmaceutical composition discussed above, wherein further the illudin analog has the following structure:

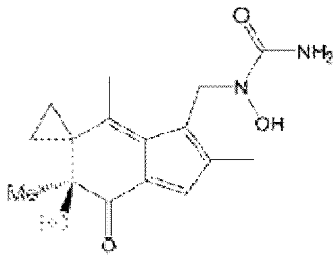
.

A method of treating cancer, the method comprises administering to a subject in need of treatment a combination of active agents comprising:

a therapeutically effective amount of an illudin or an illudin analog thereof, derivative, or a pharmaceutically acceptable salt thereof; and
a therapeutically effective amount of Ibrutinib or an analog, derivative, or a pharmaceutically acceptable salt thereof, wherein the subject has relapsed cancer and/or refractory cancer; and the subject has been treated previously with Ibrutinib; and the subject is treated with the combination after the cancer is refractory and resistant to Ibrutinib.

The pharmaceutical composition discussed above, wherein further the illudin analog is HydroxyUreaMethyl Acylfulvene.
The pharmaceutical composition discussed above, wherein further the illudin analog has the following structure:

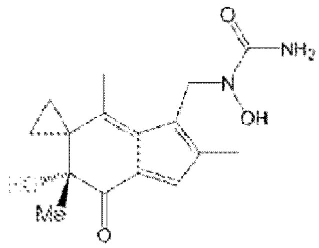
.

The pharmaceutical composition discussed above, wherein further the illudin analog is Irofulven.
The pharmaceutical composition discussed above, wherein further the illudin analog has the following structure:

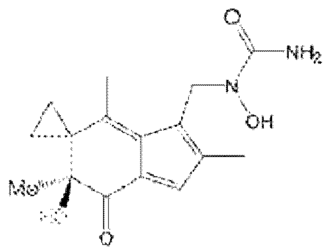
.

**Claims**

1. A pharmaceutical composition comprising a therapeutically effective amount of an illudin or an illudin analog thereof, derivative, or a pharmaceutically acceptable salt thereof; and a therapeutically effective amount of Ibrutinib or an analog, derivative, or a pharmaceutically acceptable salt thereof.

2. The pharmaceutical composition of claim 1, wherein the illudin analog is HydroxyUreaMethyl Acylfulvene.

3. The pharmaceutical composition of claim 1, wherein the illudin analog has the following structure:

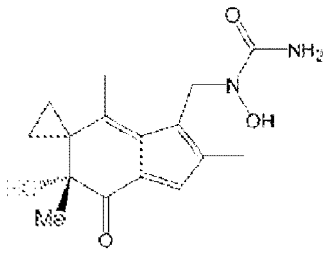

.

**4.** The pharmaceutical composition of claim 1, wherein the illudin analog has the following structure:

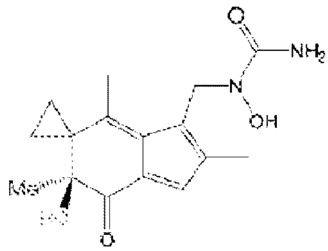

.

**5.** The pharmaceutical composition of claim 1, wherein the illudin analog is Irofulven.

**6.** A kit for the treatment of cancer in a subject comprising a therapeutically effective amount of an illudin or an illudin analog thereof, derivative, or a pharmaceutically acceptable salt thereof; and a therapeutically effective amount of Ibrutinib or an analog, derivative, or a pharmaceutically acceptable salt thereof, wherein the subject has relapsed cancer and/or refractory cancer; and the subject has been treated previously with the Ibrutinib; and the subject is treated with the combination after the cancer is refractory and resistant to Ibrutinib.

**7.** The pharmaceutical composition of claim 6, wherein the illudin analog is HydroxyU reaM ethyl Acylfulvene.

**8.** The pharmaceutical composition of claim 6, wherein the illudin analog has the following structure:

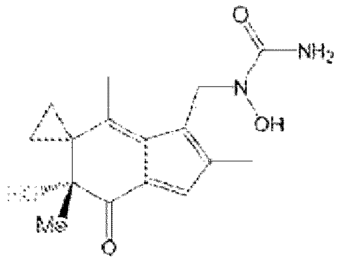

.

**9.** The pharmaceutical composition of claim 7, wherein the illudin analog is Irofulven.

**10.** The pharmaceutical composition of claim 7, wherein the illudin analog has the following structure:

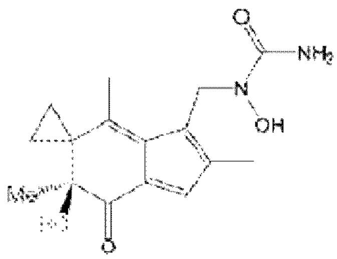

.

**11.** A composition for use in a method of treating cancer, the method comprises administering to a subject in need of

treatment a combination of active agents comprising:

a therapeutically effective amount of an illudin or an illudin analog thereof, derivative, or a pharmaceutically acceptable salt thereof; and
a therapeutically effective amount of Ibrutinib or an analog, derivative, or a pharmaceutically acceptable salt thereof,
wherein the subject has relapsed cancer and/or refractory cancer; and the subject has been treated previously with Ibrutinib; and the subject is treated with the combination after the cancer is refractory and resistant to Ibrutinib.

**12.** The pharmaceutical composition of claim 7, wherein the illudin analog is HydroxyUreaMethyl Acylfulvene.

**13.** The pharmaceutical composition of claim 7, wherein the illudin analog has the following structure:

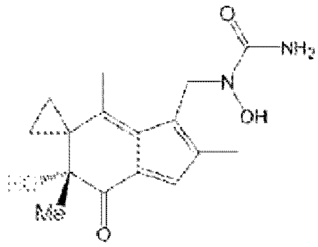

.

**14.** The pharmaceutical composition of claim 7, wherein the illudin analog is Irofulvene.

**15.** The pharmaceutical composition of claim 7, wherein the illudin analog has the following structure:

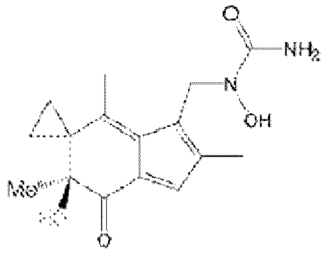

.

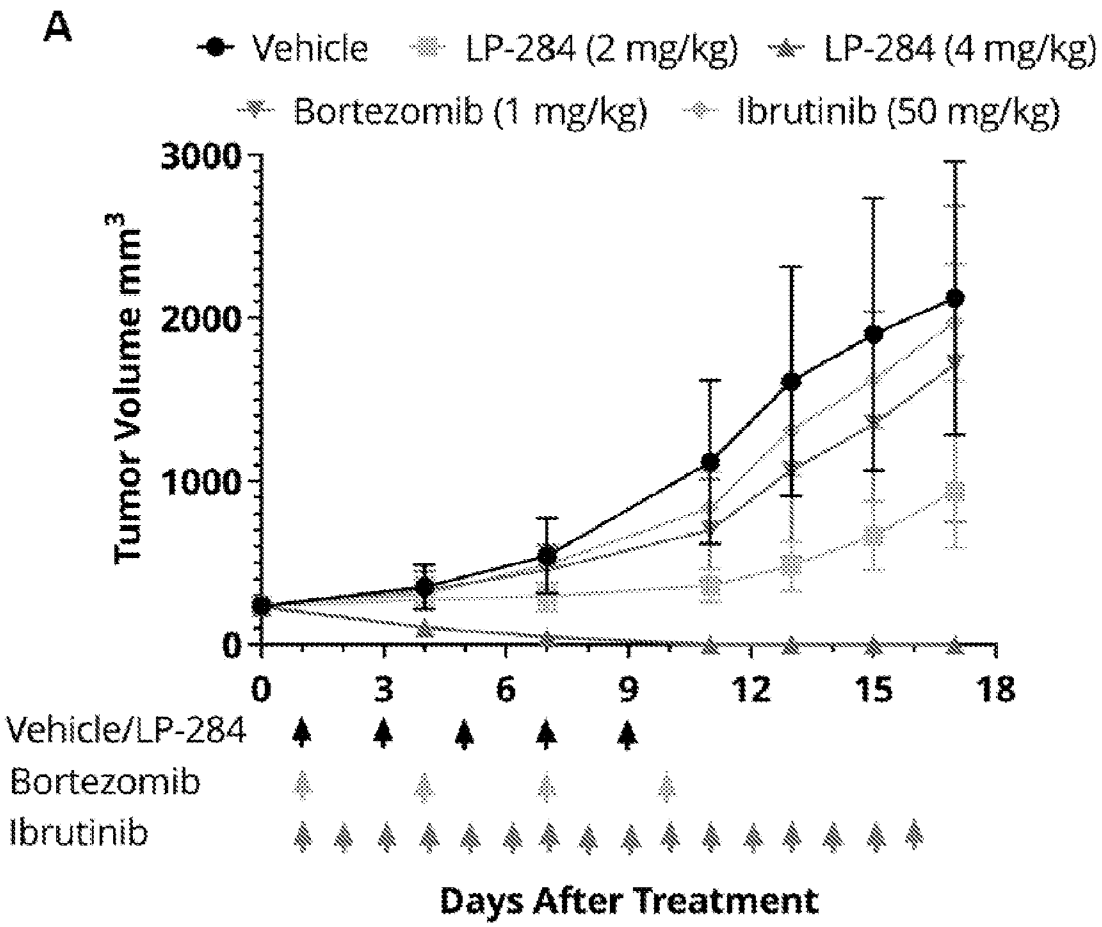
A
Vehicle
LP-284 (2 mg/kg)
LP-284 (4 mg/kg)
Bortezomib (1 mg/kg)
Ibrutinib (50 mg/kg)
Tumor Volume mm$^3$
3000
2000
1000
0
0
3
6
9
12
15
18
Vehicle/LP-284
Bortezomib
Ibrutinib
Days After Treatment

FIG. 1

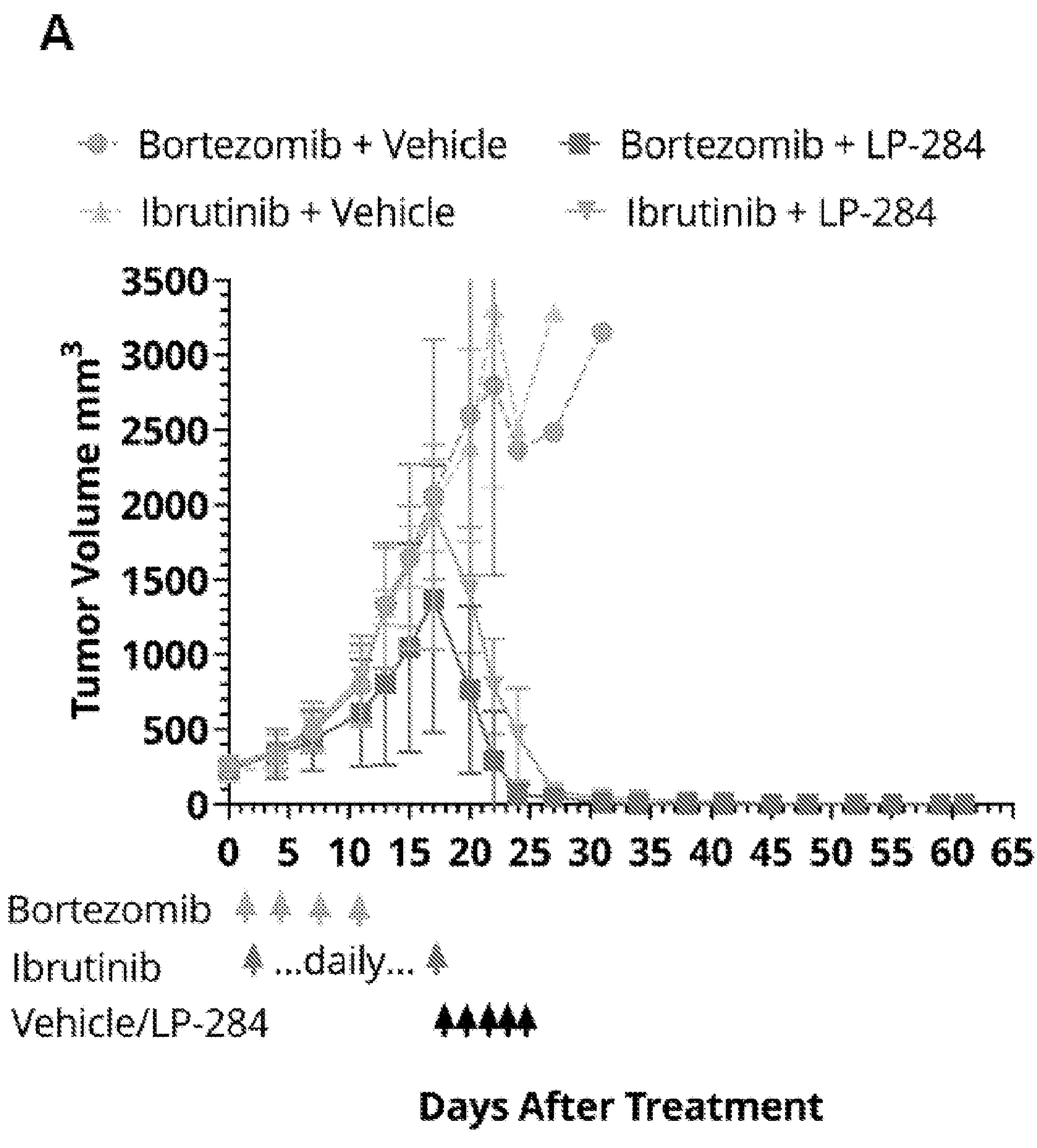
A
Bortezomib + Vehicle
Bortezomib + LP-284
Ibrutinib + Vehicle
Ibrutinib + LP-284
Tumor Volume mm3
3500
3000
2500
2000
1500
1000
500
0
0 5 10 15 20 25 30 35 40 45 50 55 60 65
Bortezomib
Ibrutinib
...daily...
Vehicle/LP-284
Days After Treatment

FIG. 2A

B

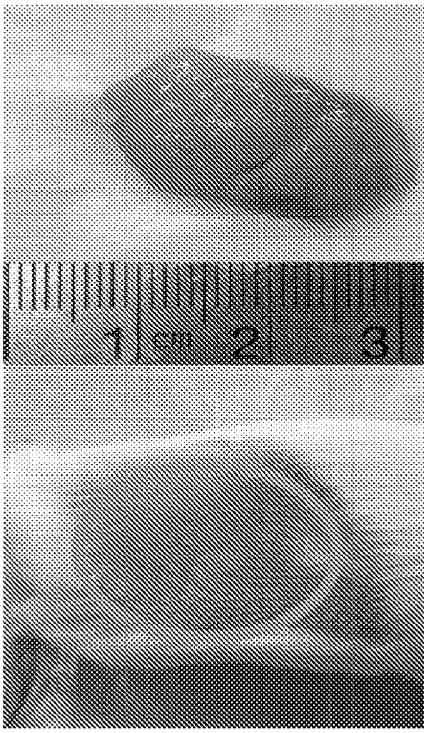


FIG. 2B

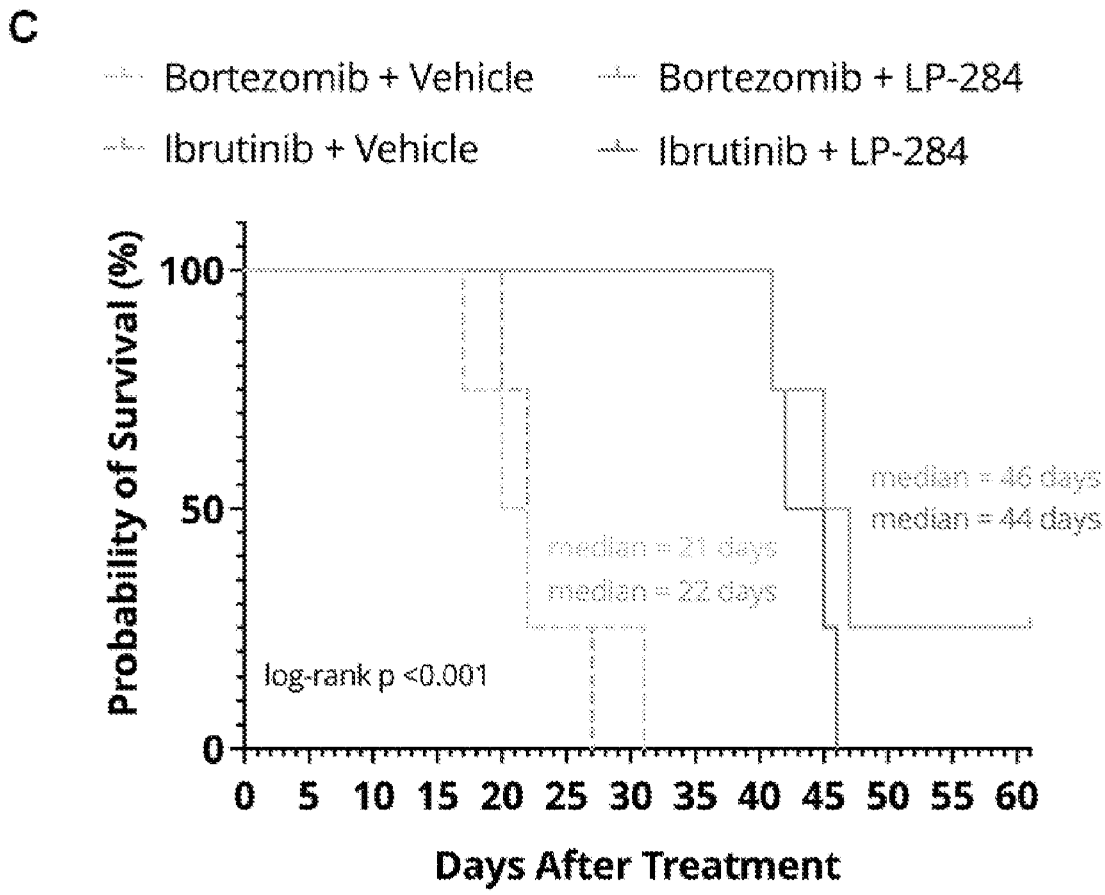
C
Bortezomib + Vehicle
Bortezomib + LP-284
Ibrutinib + Vehicle
Ibrutinib + LP-284
Probability of Survival (%)
100
50
0
median = 46 days
median = 44 days
median = 21 days
median = 22 days
log-rank p <0.001
0 5 10 15 20 25 30 35 40 45 50 55 60
Days After Treatment

FIG. 2C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016166653 A


### Non-patent literature cited in the description


- Remington: the Science and Practice of Pharmacy. Mack Publishing Co., 1995
- **LI et al.** *Proc. Natl. Acad. Sci. USA.*, 2003, vol. 100 (5), 2674-8